# EUROPÄISCHE PATENTSCHRIFT

(11) **EP 1 809 340 B1**
(45) Veröffentlichungstag und Bekanntmachung des Hinweises auf die Patenterteilung: **07.07.2010**
(21) Anmeldenummer: 05791107.5
(22) Anmeldetag: 20.09.2005
(51) Int. Cl.: A61K 49/00

(54) **STÖCHIOMETRISCH DEFINIERTE FARBSTOFFMARKIERTE SUBSTANZEN ZUR MESSUNG DER GLOMERULÄREN FILTRATIONSRATE**
STOICHIOMETRICALLY DEFINED DYE-LABELED SUBSTANCES FOR MEASURING THE GLOMERULAR FILTRATION RATE
SUBSTANCES MARQUEES AVEC DES COLORANTS DE FACON STOECHIOMETRIQUE POUR MESURER LE TAUX DE LA FILTRATION RENALE

(30) Priorität: 21.09.2004 DE 102004045748
(43) Veröffentlichungstag der Anmeldung: 25.07.2007
(73) Patentinhaber: Ruprecht-Karls-Universität Heidelberg, 69117 Heidelberg (DE)
(72) Erfinder: PILL, Johannes, 69181 Leimen (DE); KRAEMER, Uwe, 68549 Ilvesheim (DE); GRETZ, Norbert, 68259 Mannheim (DE); DEUS, Carsten, 69168 Wiesloch (DE); HAGENBRUCH, Bernd, 69469 Weinheim (DE); KLOETZER, Hans-Martin, 68163 Mannheim (DE)
(74) Vertreter: Müller-Boré & Partner Patentanwälte
(86) Internationale Anmeldenummer: PCT/EP2005/010093
(87) Internationale Veröffentlichungsnummer: WO 2006/032441

(56) Entgegenhaltungen:
- EP-A- 0 538 230
- EP-A- 0 543 333
- WO-A-01/85799
- WO-A-97/10847
- WO-A-99/31183
- LINDSTROEM K. E. ET AL.: "Physiological and Morphological effects of perfusing isolated rat kidneys with hyperosmolal mannitol solutions" ACTA PHYSIOL SCAND, Bd. 166, 1999, Seiten 231-238, XP002395607
- OHLSON M. ET AL.: "Glomerular size and charge selectivity in the rat as revealed by FITC-FIcoll and albumin" AM J PHYSIOL RENAL PHYSIOL, Bd. 279, 2000, Seiten F84-F91, XP002395608
- SOHTELL M ET AL.: "FITC-INULIN as a kidney tubule marker in the rat" ACTA PHYSIOL SCAND, Bd. 119, 1983, Seiten 313-316, XP008067913

## Beschreibung

Die Erfindung betrifft neue chemische Verbindungen, die als Markersubstanzen in der Nierendiagnostik eingesetzt werden können, ihre Herstellung und Verwendung sowie sie enthaltende Nierendiagnostika.

In der Nierendiagnostik, dort insbesondere bei der Bestimmung der glomerulären Filtrationsrate (GFR) zur Nierenfunktionsprüfung, werden - neben der so genannten "Kreatinin Clearance" (vgl. z. B. H. Burkhardt et al., Creatinine Clearance, Cockcroft-Gault-Formula and Cytastin C: Estimators of True Glomerular Filtration Rate in the Elderly?, Gerontology 48 (2002) 140-146) und der Verwendung von radioaktiv markierten Kontrastmitteln (vgl. z. B. B. Frennby et al., Contrast media as Markers for GFR, Eur. Radiol. 12 (2002) 475-484) - unter anderem Fructane als Markersubstanzen beschrieben. Fructane, die auch als Polyfructosane bezeichnet werden, sind Oligo- und Polysaccharide, die aus geraden oder verzweigten Fructoseketten aufgebaut sind, welche auf ein Grundmolekül Saccharose aufgepfropft sind. Je nach Grad der Verzweigung der Fructoseketten und je nach Grad der Polymerisation können unterschiedliche Fructane unterschiedliche physikalische Eigenschaften aufweisen, beispielsweise unterschiedliche Wasserlöslichkeiten. Viele Fructane kommen in Pflanzen als Reservekohlenhydrate vor, beispielsweise in den unterirdischen Teilen von Compositen, Campanulaceen, Gräsern und Liliaceen.

In der Nierenfunktionsprüfung werden insbesondere die Fructane Inulin und Sinistrin als Markersubstanzen eingesetzt. Inulin und Sinistrin sind aus jeweils ca. 10 bis 40 Fructoseeinheiten aufgebaut und weisen dementsprechend Molekulargewichte von ca. 1600 bis ca. 6500 auf. Sowohl Inulin als auch Sinistrin werden nach parenteraler Applikation weder durch den Stoffwechsel verändert noch im Organismus gespeichert, sondern durch die Nierenglomeruli ausfiltriert und in den Tubuli nicht wieder rückresorbiert.

Zur Beurteilung der Nierenfunktion wird üblicherweise der zeitliche Verlauf der Markersubstanzkonzentration im Blut nach parenteraler Gabe einer bestimmten Dosis der Markersubstanz bestimmt. Die Bestimmung der Konzentration der Markersubstanz im Blut kann beispielsweise mittels enzymatischer Methoden erfolgen (vgl. z. B. H.F. Kuehnle et al., Fully enzymatic inulin determination in small volume samples without deproteinization, Nephron 62 (1992) 104 - 107). Im Fall von Inulin als Markersubstanz wird unter anderem auch die Möglichkeit beschrieben, mit einem Fluoreszenzmarker versehenes Inulin, beispielsweise Fluoresceinisothiocyanat-markiertes Inulin (FITC-Inulin), zu verwenden und die Konzentration der Markersubstanz mittels Fluoreszenzmessung zu bestimmen (vgl. z. B. M. Sohtell et al., FITC-inulin as a kidney tubule marker in the rat, Acta Physiol. Scand. 119 (1983) 313-316; J. N. Lorenz & E. Gruenstein, A simple, nonradioactive method for evaluating single-nephron filtration rate using FITC-inulin, Am. J. Physiol. 276 (Renal Physiol. 45) (1999) F172-F177).

Inulin und FITC-Inulin haben für die klinische Alltagsroutine den Nachteil, daß sie in Wasser nur sehr gering löslich sind und in wässrigen Zubereitungen bei der Lagerung auskristallisieren. Vor der Applikation müssen die inulinhaltigen Zubereitungen deshalb in der Regel erwärmt werden, um das Inulin oder FITC-Inulin wieder in Lösung zu bringen. Durch diese Maßnahme wird das Inulin jedoch je nach Dauer des Erhitzens hydrolytisch angegriffen und teilweise bis zur Fructose abgebaut. Zudem verbleiben bei unvollständigem Auflösen Reste von nicht gelösten Inulinteilchen in der Zubereitung, die nur schwer zu erkennen sind und die nach einer Injektion schwere Kreislaufkomplikationen zur Folge haben können. Die geringe Löslichkeit von Inulin oder FITC-Inulin führt dazu, daß eine definierte Konzentration der Markersubstanz in einer Injektionslösung nur schwer zu erreichen ist. Außerdem hat die Anwendung von Inulin oder FITC-Inulin nach der Injektion in das Versuchstier einen transienten Blutdruckabfall zur Folge. Diese Kreislaufreaktion dauert in günstigen Fällen 5 Minuten. Insbesondere wird durch den Kreislaufeinbruch gerade die zu bestimmende Nierenfunktion gestört.

Auch Sinistrin wird als Markersubstanz in der Nierendiagnostik eingestezt (vgl. z. B. W. Estelberger et al., Determination of Glomerular Filtration Rate by Indentification of Sinistrin Kinetics, Eur. J. Clin. Chem. Clin. Biochem. 33 (1995) 201-209), obschon auch für Sinistrin Nebenwirkungen beschrieben sind (vgl. hierzu auch R. Chandra et al., Anaphylactic Reaction to Intravenous Sinistrin (Inutest), Ann. Clin. Biochem. 39 (2002) 76). Sinistrin ist wie Inulin ein Fructan und kann durch Extraktion aus fructanhaltigen Pflanzenteilen gewonnen werden (vgl. z. B. EP-B 0 568 574). Die Verwendung von Sinistrin als Markersubstanz erfordert jedoch verhältnismäßig hohe Sinistrinkonzentrationen in den entsprechenden Zubereitungen, die im Bereich von 100 mg je kg Körpergewicht des zu untersuchenden Individuums liegen, da Sinistrin selbst nur in Blutproben bestimmt werden kann, und die hierfür zur Verfügung stehenden Analysenmethoden verhältnismäßig unempfindlich sind Zudem ist der Nachweis von Sinistrin nur mit einer mehrstufigen enzymatischen Reaktion möglich, bei der zunächst nach Entfernen der endogenen Blutglucose das Sinistrin in Glucose umgewandelt und die so erhaltene Glucose als Maß für das Sinistrin bestimmt wird. Erfahrungsgemäß sind solche mehrstufigen Reaktionen aufwendig und oft mit einem nicht unerheblichen Fehler behaftet.

Aus WO 99/31183 und WO 01/85977 ist es bekannt, durch Kopplung von Polyfructosanen mit Farbstoffen Substanzen zu erhalten, die zur GFR-Bestimmung einsetzbar sind.

WO 01/85977 beschreibt insbesondere die Kopplung von Sinistrin an den Fluoreszenzfarbstoff FITC. FTTC-Sinistrin kann in geringeren Dosen als Sinistrin in der Nierendiagnostik eingesetzt werden. Typischerweise werden Dosen von 5 bis 50 mg/kg Körpergewicht (bevorzugt ca. 5 bis 30 mg/kg Körpergewicht) verabreicht. Nicht-invasive, auf Fluoreszenzdetektion basierende Nachweismethoden erlauben die empfindliche und zuverlässige Detektion von FITC-Sinistrin in der Nierenfunktionsdiagnostik.

WO 02/05858 beschreibt Reagenzien für die Bestimmung der GFR, die mit elektrochemilumineszenten Gruppen konjugierte Polyaminopolyessigsäurederivate enthalten. Die elektrochemilumineszente Gruppen enthalten vorzugsweise Lanthanoidionen. Die Reagenzien sind dabei für transkutane Messungen geeignet.

WO-A-97/10847 beschreibt ein Verfahren zur Prävention und Behandlung von insulinabhängiger Diabetes mellitus, welches *inter alia* hydrophile Polymere einsetzt.

EP-A-0 538 230 beschreibt ein Verfahren zur Herstellung von synthetischen N-gebundenen Glykokonjugaten.

Die Nachteile des Standes der Technik können wie folgt zusammengefasst werden:
1.) Die Kreatinin-Clearance ist sehr ungenau und hängt stark von Ernährung und körperlicher Tätigkeit ab.
2.) Der Einsatz von radioaktiven Isotopen erfährt in der Humanmedizin verständlicherweise eine große Ablehnung.
3.) Beim Einsatz der natürlichen Polymere Inulin und Sinistrin kann es zu anaphylaktischen Reaktionen bei intravenöse Gabe kommen. Insbesondere die nicht-farbstoffmarkierten Verbindungen müssen in hohen Dosen verabreicht werden.
4.) Die enzymatische Analytik beim Einsatz von Sinistrin oder Inulin sind arbeits- und zeitaufwändig.
5.) Bei FITC-markiertem Inulin und Sinistrin handelt es sich um nicht stöchiometrisch genau definierte Verbindungen: zum einen sind Inulin und Sinistrin aus natürlichen Rohstoffen gewonnene Polymere mit stark variierenden Kettenlängen; zum anderen kann die FITC-Markierung an beliebige Hydroxygruppen der Fructose-Untereinheiten von Inulin und Sinistrin gekoppelt sein; weiterhin ist der Belegungsgrad, das heißt das Verhältnis FITC zu Sinistrin bzw. Inulin nur statistisch und daher sehr ungenau ermittelbar. Gerade für Substanzen, die als in-vivo-Diagnostikum eingesetzt werden sollen, ist es jedoch wünschenswert, die Substanzen möglichst rein und in möglichst reproduzierbarer Weise bereitstellen zu können.
6.) Nachteile der in WO 02/05858 offenbarten Verbindungen sind die in-vivo-Verwendung von physiologisch bedenklichen Komplexbildnern sowie die Verabreichung von toxischen Schwermetallen (Lanthaniden).

Aufgabe der vorliegenden Erfindung ist es daher, die Nachteile des Standes der Technik zu beseitigten. Insbesondere ist es die Aufgabe der vorliegenden Erfindung, Substanzen bereitzustellen, die als Markersubstanzen in der Nierenfunktionsprüfung eingesetzt werden können und die gegenüber den im Stand der Technik bekannten Markersubstanzen, insbesondere Inulin, FITC-Inulin, Sinistrin und FITC-Sinistrin, Vorteile aufweisen.

Die Aufgabe wird durch den Gegenstand der Erfindung, wie er in den Patentansprüchen angegeben ist, gelöst.

Gegenstand der Erfindung sind farbstoffmarkierte Substanzen, das heißt Verbindungen der allgemeinen Formel I

P-Lₘ-F (I)

in der P ein Polyol, L einen Linker und F einen Farbstoffrest symbolisieren.

P ist dabei ein Polyol, das aus der Gruppe ausgewählt ist umfassend: Glycerin, Mannitol, Sorbitol, Hexite, Pentite, Tetirite, Inosite, Mannose, Aldosen, Laktose, Gentiobiose, β-Alkylglylkoside, Desoxyzucker, β-Alkyluronsäuren, Fucose, Desoxyzuckeralkohole, sowie jeweils Derivaten hiervon. "Derivat" bedeutet dabei, dass das Polyol auch als Desoxyaminozuckeralkohol vorliegen kann.

L ist dabei eine Linkergruppe, die aus der Gruppe ausgewählt ist, bestehend aus Thioharnstoff-Gruppe (-N-CS-N-), Thiocarbamat-Gruppe (-N-CS-O-), Carbamat-(Urethan-)-Gruppe (-N-CO-O-), Ether-Gruppe (-O-), Thioether-Gruppe (-S-), Ester-Gruppe (-CO-O-), Amid-Gruppe (-CO-N-), Thioester-Gruppe (-CS-O-), Thioamid-Gruppe (-CS-N-), Aminoalkyl-Gruppe (-CO-N-(CH₂)ₙ-O-) mit n = 2 bis 5, sekundäre Amin-Gruppe (-NH-). Der Parameter m ist dabei 1.

F ist dabei ein Farbstoff, insbesondere ein Fluoreszenzfarbstoff, der aus der Gruppe ausgewählt ist umfassend:
Fluorescein- Farbstoffe, Cyanin-Farbstoffe, Naphthylamid-Farbstoffe, Coumarin-Farbstoffe, Xanthen-Farbstoffe, Thioxanthen-Farbstoffe, Naphtholacton-Farbstoffe, Azlacton-Farbstoffe, Methin-Farbstoffe, Oxazin-Parbstoffe, Thiazin-Parbstoffe. Bevorzugt ist F ein Fluorescein-Farbstoff, besonders bevorzugt Fluorescein.

Die erfindungsgemäße Lösung der Probleme des Standes der Technik sieht vor, dass Polyole als glomerulär filtrierbare Substanzen (d. h. Substanzen mit ausreichender Hydrophilie) mit definierter Struktur im stöchiometrischen Verhältnis 1:1 mit einem Farbstoff, insbesondere einem Fluoreszenzfarbstoff wie beispielsweise FITC, gekoppelt werden. Durch Variation der glomerulär filtrierbaren Substanz bezüglich ihrer Polarität erreicht man eine Beeinflussung der Polarität des gesamten Farbstoff-Polyol-Konjngats, womit die glomeruläre Filtzierbarkeit exakt eingestellt werden kann. Durch den genau definierten Labelling-Grad (d. h. das stöchiometrisch einstellbare 1:1-Verhältnis von Polyol zu Farbstoff) kann eine deutlich geringere und damit wesentlich verträglichere Dosierung in der Applikation am Patienten und eine deutlich erleichterte Handhabung im Umgang mit den Lösungen erreicht werden. Außerdem wird durch die Bereitstellung der erfindungsgemäßen Substanzen und der Verfahren zu deren Herstellung eine hohe Reproduzierbarkeit und Chargenhomogenität garantiert und so die Einhaltung der geforderten Spezifikationen für Arzneimittel ermöglicht.

Die Substanzen der vorliegenden Erfindung sind erhältlich durch Umsetzung von Polyolen P oder deren Derivaten mit entsprechenden Farbstoffen F, wobei die Kopplung zum Polyol-Farbstoff-Konjugat mittels entsprechender Linker L bewirkt wird. Bevorzugte Darstellungsvorschrilften für FITC-Mannose sind in dem Beispiel 2 angegeben.

Weiterhin ist Gegenstand der Erfindung die Verwendung von der erfindungsgemäßen farbstoffmarkierten Substanzen als Bestandteil einer diagnostischen Zubereitung, die insbesondere für die Nierendiagnostik geeignet ist, sowie ein Diagnostikum, insbesondere eine diagnostische Zubereitung, das die erfindungsgemäßen farbstoffmarkierten Substanzen enthält.

Die erfindungsgemäßen farbstoffmarkierten Substanzen werden bevorzugt als Bestandteil von parenteral zu verabreichenden Zubereitungen für die Nierenfunktionsprüfung verwendet. Zur Herstellung des Diagnostikums werden die erfindungsgemäßen farbstoffmarkierten Substanzen in *aqua ad inj*. (Wasser für Injektionszwecke gemäß DAB 10) oder physiologischer Kochsalzlösung (isotonische Natriumchloridlösung) gelöst. Die Konzentrationen der entsprechenden farbstoffmarkierten Substanzen in den diagnostischen Zubereitungen liegen im Bereich von 0,350 mg/ml bis 0,9 mg/ml. Neben der entsprechenden farbstoffmarkierten Substanz kann das parenteral zu applizierende Diagnostikum auch noch physiologisch verträgliche Puffersubstanzen enthalten.

Die Anwesenheit der Farbstoffgruppe, insbesondere der Fluorescein-Gruppe, in den erfindungsgemäßen farbstoffmarkierten Substanzen ermöglicht deren Bestimmung anhand von optischen Messungen, insbesondere anhand von Fluoreszenzmessungen. Diese können in vitro durchgeführt werden, beispielsweise in Blutproben. Für die Fluoreszenzmessung, in beispielsweise Blut-, Serum- oder Plasmaproben, ist keine enzymatische Vorbehandlung der Blutprobe notwendig. Zudem bietet die Fluoreszenzmessung den Vorteil hoher Empfindlichkeit und Schnelligkeit der Messung. Die Messung kann mit üblichen Standardgeräten durchgeführt werden. Der Einsatz der erfindungsgemäßen farbstoffmarkierten Substanzen als Markersubstanzen in der Nierendiagnostik erlaubt auch nicht-invasive Nachweismethoden. Nicht-invasive Nachweismethoden sind im hier benutzten Sprachgebrauch Methoden, die den Nachweis einer Substanz in Gewebe oder Körperflüssigkeiten ohne vorherige Probennahme, beispielsweise durch Blutabnahme nach einer Venenpunktion oder durch Gewinnung von Kapillarblut aus der Fingerbeere oder dem Ohrläppchen, erlauben.

Vorzugsweise wird als nicht-invasives Verfahren zur Bestimmung der erfindungsgemäßen farbstoffmarkierten Substanzen im Gewebe oder in Körperflüssigkeiten ein Fluoreszenzmessverfahren eingesetzt, bei dem Licht zur Anregung der Fluoreszenz in die Haut des zu untersuchenden Individuums eingestrahlt wird und das aus der Haut austretende Licht, insbesondere Fluoreszenzlicht, detektiert wird. Vorteilhafterweise kann dies mit Hilfe eines nicht-invasiven Messkopfes geschehen, bei dem eine Lichtquelle, beispielsweise ein im UV-Bereich emittierender Laser oder eine LED, über eine Glasfaseroptik die Haut beleuchtet und die darin enthaltenen erfindungsgemäßen, mit einem Fluoreszenzfarbstoff markierten Substanzen zur Fluoreszenz anregt. Das Fluoreszenzlicht wird mit einer Glasfaseroptik aufgenommen und mit einem entsprechenden Detektor, beispielsweise einem CCD-Spektrographen oder einem Photodetektor mit vorgeschaltetem Bandpassfilter, gemessen. Die Lichtquelle und/oder der Detektor können dabei in den Messkopf integriert oder außerhalb des Messkopfes angeordnet sein. Der Messkopf wird mit einem transparenten Kleber, beispielsweise einer transparenten Klebefolie, auf die Haut des zu untersuchenden Individuums aufgeklebt und verbleibt dort für die gesamte Messzeit. Ein entsprechendes Verfahren ist ein weiterer Gegenstand der vorliegenden Erfindung.

Da die Bestimmung der erfindungsgemäßen farbstoffmarkierten Substanzen mit Hilfe empfindlicher Fluoreszenzmessungen möglich ist, kann die Substanz-Menge, die dem zu untersuchenden Individuum verabreicht wird, deutlich niedriger sein, als dies mit den im Stand der Technik beschriebenen Substanzen, insbesondere Inulin, FITC-Inulin, Sinistrin und FITC-Sinistrin, der Fall ist. Während für Sinistrin Dosen von 100 mg Substanz je kg Körpergewicht des zu untersuchenden Individuums notwendig sind und für FITC-Sinistrin immerhin noch Dosen von 5 bis 50 mg erforderlich sind, können die erfindungsgemäßen Substanzen bereits bei weniger als 1 mg je kg Körpergewicht des zu untersuchenden Individuums nachgewiesen werden. Durch die niedrige Dosierung wird eine Belastung des zu untersuchenden Organismus' im Vergleich zu den bisher bekannten Substanzen deutlich vermindert. Das niedrige Applikationsvolumen und die niedrigere Dosis, mit der die erfindungsgemäßen farbstoffmarkierten Substanzen eingesetzt werden können, wird ermöglicht durch deren hohe Löslichkeit und das stöchiometrische Farbstoff: Polyol-Verhältnis (1:1). Zudem sind die Ausgangsstoffe für die erfindungsgemäßen Substanzen, insbesondere die darin verwendeten Polyole, chemisch eindeutig definiert erhältlich (anders als dies zum Beispiel für die Naturstoffe Inulin oder Sinistrin möglich ist), was vor allem der Reproduzierbarkeit der Substanzsynthese zuträglich ist.

Zudem ist eine nicht-invasive Detektion der fluoreszenzgelabelten erfindungsgemäßen Substanzen möglich. Dies trägt ebenfalls zur Reduzierung der körperlichen Beeinträchtigung des zu untersuchenden Individuums bei, da keine Blutproben für die Untersuchung und Bestimmung genommen werden müssen.

Die nicht-invasive Messung des Gehalts an den erfindungsgemäßen farbstoffmarkierten Substanzen kann kontinuierlich über einen längeren Zeitraum, beispielsweise über die klinisch relevante Messzeit von 180 min für die Nierenfunktionskontrolle (GFR), erfolgen. Dies trägt zu einer präzisen Diagnostik bei.

Bislang wurden bei der Verwendung der erfindungsgemäßen farbstoffinarkierten Substanzen als Markersubstanzen für die Nierenfunktionsprüfung keine unerwünschten Kreislaufreaktionen bei den untersuchten Individuen festgestellt. Die glomeruläre Filtrationsrate kann somit ohne sekundären Einfluss auf die Niere bestimmt werden. Dies ist ein deutlicher Vorteil gegenüber der bekannten Verwendung von FITC-Inulin oder Inulin bzw. FITC-Sinistrin und Sinistrin.

Die vorliegenden Erfindung wird durch die nachfolgenden Beispiel und Figuren näher erläutert:
Figur 1 zeigt die Strukturformel für Fluoresceinisothiocyanat-Cellobiose (FITC-CEL).
Figur 2 zeigt die Strukturformel für Fluoresceinisothiocyanat-Mannose (FITC-MAN).
Figur 3 zeigt die Strukturformel für Fluoresceinisothiocyanat-Hexaethylenglykol (FITC-HEG).
Figur 4 zeigt die mittleren Konzentrationsverläufe (in ng/ml) für FITC-HEG (im Diagramm als FITC-PEG bezeichnet) über die Zeit (time) in Minuten vor (gefüllte Quadrate) und nach (offene Quadrate) unilateraler Nephrektomie in Blutproben von Versuchstiere.
Figur 5 zeigt die mittleren Konzentrationsverläufe (in ng/ml) für FITC-MAN (im Diagramm als FITC-MA bezeichnet) über die Zeit (time) in Minuten vor (gefüllte Kreise) und nach (offene Kreise) unilateraler Nephrektomie in Blutproben von Versuchstieren.
Figur 6 zeigt die mittleren Konzentrationsverläufe (in ng/ml) für FITC-CEL (im Diagramm als FITG-Cellubiose bezeichnet) über die Zeit (time) in Minuten vor (gefüllte Dreiecke) und nach (offene Dreiecke) unilateraler Nephrektomie in Blutproben von Versuchstieren.

### Beispiel 1: (Vergleichsbeispiel)

### 1.1. Herstellung von Cellobiosamin

Die Herstellung von Cellobiosamin erfolgt im Wesentlichen gemäß J. Carbohydr. Chem. 1992, 11(7), 813-835.

1-Benzylamino-1-desoxy-4-O-beta-d-glucopyranosyl-d-glucitol:d-Cellobiose (1 g, 2.9 mmol) wird in 1 ml Wasser gelöst Die Lösung erwärmt man auf 60°. Bei dieser Temperatur tropft man Benzylamin (0.5 ml, 4.6 mmol) hinzu (ca. 3 Minute). Nach weiteren 15 Minuten bei 60° wird die Lösung klar. Man erwärmt noch 3 Stunden, lässt etwas abkühlen (50°), gibt 4 ml Methanol hinzu und lässt dann auf RT abkühlen. Im Wasserbad (22°) gibt man nach und nach Natriumborhydrid (0.23 g, 6.1 mmol) hinzu und rührt anschließend noch 36 Stunden bei Raumtemperatur. Man verdünnt mit Wasser und Methanol, zieht das Methanol ab und extrahiert die wäßrige Lösung zweimal mit Ether. Die wäßrige Lösung wird mit 3 n HCl auf pH 3 gebracht, zweimal mit Methanol eingeengt, wieder mit Methanol aufgenommen, filtriert und zum Sirup eingedampft.

Der Sirup wird mit 25 ml Wasser verdünnt und mit 25%iger Ammoniaklösung auf pH 9 gestellt. Pd/C (10%, 0.3 g) wird zugegeben, und unter kräftigem Rühren hält man 24 Stunden unter Wasserstoffatmosphäre. Dünnschichtchromatographie (DC) (PAW 30/6/12) zeigt, dass das Edukt verschwunden ist (RF,Produkt = 0.1; RF,Edukt = 0.47). Man trennt den Katalysator ab, wäscht mit Wasser nach und schüttelt die wässrige Lösung mit Essigester aus. Nach dem Einengen verbleiben 2.2 g eines halbfesten, amorphen Feststoffs. DC (PAW 30/12/18): RF, Produkt 0.33, Nebenprodukte mit RF 0.36 (Ninhydrin-positiv), RF 0.48 (nur Permanganat-positiv). Man erhält geschätzt >90% an Produkt.

Zur Reinigung und Abtrennung von Salzen reinigt man über IR-120(H+). Dazu werden ein Fünftel der wässrigen Lösung des Rohprodukts (entspricht etwa 0.6 mmol, ∼200 mg) auf eine IR-120(H+)-Säule (5 x 1.6 cm, entspricht etwa 19 mmol) aufgebracht. Man spült mit ca. 60 ml Wasser und eluiert dann mit 0.25 m NH3-Lösung (ohne Gradient). Man erhält zwei Fraktionen, die im DC praktisch einheitlich Produkt zeigten. Der Nebenfleck mit RF 0.48 befindet sich in der ersten Fraktion, der sehr blasse Nebenfleck mit RF 0.36 kann nicht mehr detektiert werden. Auswaage an Produkt ca. 0.10 g (entsprechend über drei Stufen ca. 50%).

### 1-H-NMR entspricht der Literatur.

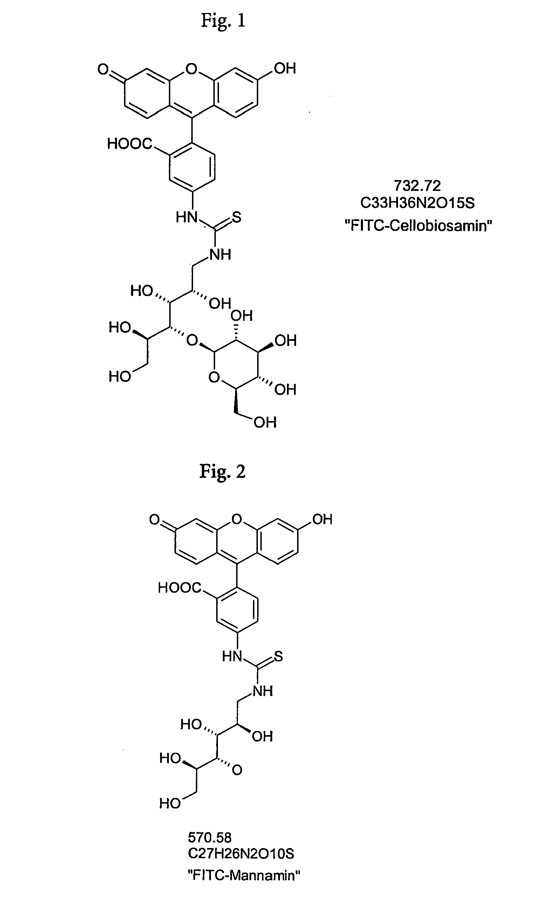

### 1.2. Herstellung von Fluoresceinisothiocyanat-Cellobiose (FITC-CEL; Figur 1)

Zu ca. 15 mg Cellobiosamin (0.044 mmol; 1-Amino-1-desoxy-4-O-β-D-glucopyranosyl-D-glucitol) in 1 ml Wasser und 0.5 ml Methanol gibt man 0.05 ml Triethylamin (0.36 mmol) und versetzt anschließend bei 0° portionsweise mit insgesamt 33 mg FITC-Hydrochlorid (99%ig, 0.077 mmol, Acros). Das Dünnschichtchromatogramm (mit einem n-Propanol/Ammoniak/Wasser-Gemisch im Verhältnis 10:1:3 als Laufmittel) zeigt Produkt, unpolare Zersetzungsprodukte des FITC, geringe Nebenprodukte in der Nähe des Produkts, aber kein Edukt-Amin mehr. Man engt die Reaktionsmischung im Vakuum ein, dampft mit Wasser nach (die Lösung hat dann pH 7-8) und stellt mit Essigsäure vorsichtig auf pH 4. Die wässrige Lösung wird dreimal mit Essigester extrahiert. Das Dünnschichtchromatogramm der wässrigen Phase zeigte nun zu etwa 95 % FITC-CEL. Man rotiert ein, dampft zweimal mit Wasser nach (dito am Rotationsverdampfer im Vakuum) und lyophilisiert dann zweimal, um eventuelle Triethylamin- und Essigsäure-Spuren zu beseitigen. Als Auswage erhält man 10 mg (32 %) FITC-CEL.

### Beispiel 2:

### 2.1. Herstellung von Mannamin

0.178 mol Hydroxylamin (freigesetzt aus 0.178 mol = 12.36 g Hydroxylamin-Hydrochlorid mit alkoholischer Natriumalkoholat-Lösung) in ca. 200 ml Ethanol werden auf 75° erwärmt und portionsweise mit 0.1 mol = 18.0 g d-Mannose versetzt. Man hält noch eine halbe Stunde bei dieser Temperatur, lässt dann über Nacht abkühlen und saugt den kristallinen Niederschlag ab. Nach Waschen mit 50 ml Ethanol und Trocknen im Vakuum erhält man 18.7 g (96%) d-Mannose-Oxim, Schmelzpunkt 173°(Zers.).

### 2.14 g d-Mannose-Oxim in 21 ml Eisessig werden mit 80 mg Platinoxid 24 Stunden bei

Raumtemperatur hydriert (H2-Gas, ca. sechs Stunden vor Beendigung hört die Wasserstoffaufnahme auf). Man filtriert, wäscht mit reichlich Essigsäure und Wasser und engt das Filtrat ein. Das so erhaltene "Mannammonium"-Acetat lässt sich nicht kristallisieren; Dünnschichtchromatogramm (DC) (Butanol/Eisessig/Wasser = 4:1:2): ein unpolarer Nebenspot (Ninhydrin- und Permanganat-positiv) und ein polarer Nebenspot (nur mit Permanganat Anfärbung), ca. 1-2% bzw. 3-5%.

Zur Entfernung der Essigsäure und eventuellen Abtrennung der Nebenprodukte reinigt man über einen sauren Ionenaustauscher (IR-120(H+); Säule: 2.2 x 9 cm, Volumen ca. 35 ml = 65 mÄq.). Man eluiert mit 80 ml Wasser und dann mit verdünnter wässriger Ammoniaklösung, Gradient von 0.3 n bis 0.5 n. Die Produktfraktionen werden vereint und eingeengt, Reinheit nach DC-Schätzung etwa 99%. Das Produkt Mannamin verbleibt als blassgelber Sirup, 280 mg (70%).

### 2.2. Herstellung von Fluoresceinisothiocyanat-Mannose (FITC-MAN; Figur 2)

Ca.120 mg (0.7 mmol) Mannamin in einer Mischung aus 1.5 ml Wasser und 3.5 ml Methanol werden bei Raumtemperatur unter kräftigem Rühren nach und nach mit kleinen Portionen (jeweils etwa 5 mg) FITC-Hydrochlorid (99%ig, Acros) versetzt. Nach Zugabe von etwa 100 mg FITC (0.257 mmol) liegt der pH-Wert der Lösung bei 8-9 (am Anfang ist die Lösung sehr basisch), und man versetzt mit 48 mg (0,35 mmol) Kaliumcarbonat. Nun wird weiter FITC in kleinen Portionen zugegeben. Den Fortgang der Reaktion verfolgt man per Dünnschichtchromatographie (DC) und pH-Messung. Laufmittel für die DC ist ein Essigester/Methanol/Wasser/Essigsäure-Gemisch im Verhältnis 120/20/1/1. Der RF-Wert von FITC-MAN ist 0,38; die RF-Werte der Nebenprodukte sind 0,80 und 0,88.

Nach Zugabe von insgesamt 185 mg (0.48 mmol) FITC beendet man die Reaktion. Das Dünnschichtchromatogramm zeigt Produkt und Nebenprodukte im Verhältnis von etwa 10:1, alle Spots sind stark gelb gefärbt und lassen sich außerdem gut mit Kaliumpermanganat-Lösung anfärben. Man zieht das Methanol im Vakuum ab, verdünnt mit Wasser und stellt mit 1N HCl auf pH 6-7. Das Volumen der wässrigen Lösung beträgt 15 ml. Man wäscht zweimal mit jeweils 10 ml Essigester, verwirft die organischen Phasen und stellt dann die wässrige Phase mit 1N HCl vorsichtig auf pH 4-5. Daraufhin wird dreimal mit jeweils 10 ml Essigester extrahiert. Die nach DC-Prüfung vereinigten Essigesterphasen werden zur Trockne eingeengt, mit Wasser ausgenommen und lyophilisiert. Anschließend trocknet man im Vakuum über Phosphorpentoxid. Die DC-Kontrolle zeigt einheitliches Produkt. Die Auswaage an FITC-MAN beträgt 110 mg (41 % bezogen auf FITC).

### Beispiel 3: (Vergleichsbeispiel)

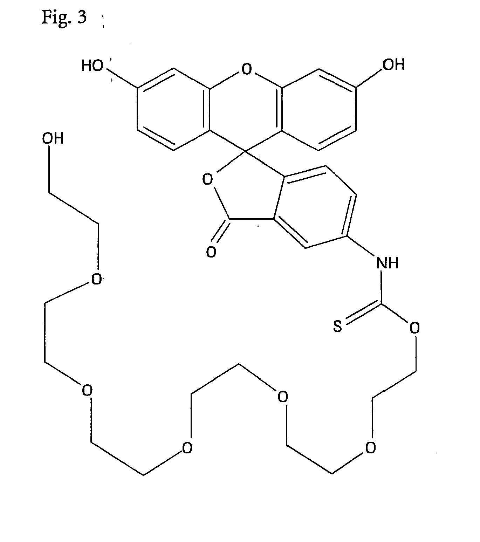

### Herstellung von Fluoresceinisothiocyanat-Hexaethylenglykol (FITC-HEG; Figur 3)

Zu einer Lösung von 2,6 g Hexaethylengtykol (Aldrich) in 30 ml DMF werden in einer Stickstoffatmosphäre bei Raumtemperatur 15 g NaH (60 % Dispersion in Mineralöl) portionsweise zugegeben. Dabei steigt die Temperatur auf ca. 40°C. Die resultierende Lösung wird für 30 min. bei dieser Temperatur belassen. Anschließend werden 3,0 g Fluoresceinisothiocyanat Hydrochlorid (99%ig, Acros, gelöst in 80 ml DMF) zugetropft. Die Lösung wird bei 40°C für 1 h und anschließend bei Raumtemperatur über Nacht geführt. Nach Abkühlen auf 5°C wird langsam eine Lösung von 20 g NH₄Cl in 100 ml H₂O zugegeben. Die Lösungsmittel werden im Vakuum abgezogen und der Rückstand wird in 80 ml Methanol gelöst. Nach Zugabe von 100 ml Kieselgel-Standardsäulenmaterial als Adsorptionsmittel wird das Lösungsmittel verdampft. Der Rückstand wird mit einem Laufmittel aus einem Ethylacetat/Methanol-Gemisch (8:2) mittels einer Silica-Säule chromatographiert. Wiederholte Chromatographie liefert 2,0 g Rohprodukt. Das Rohprodukt wird in 200 ml eines t-Butanol / H₂O-Gemisches (1:1) gelöst und gefriergetrocknet. Man erhält 1,3 g eines orangefarbenen Feststoffs (FITC-HEG).

### Beispiel 4

### Bestimmung der glomerulären Filtrationsrate durch Fluoreszenzmessung der FITC-markierten Substanzen aus Beispiel 1 bis 3 im Tierversuch

### 1. Untersuchungen in der Ratte

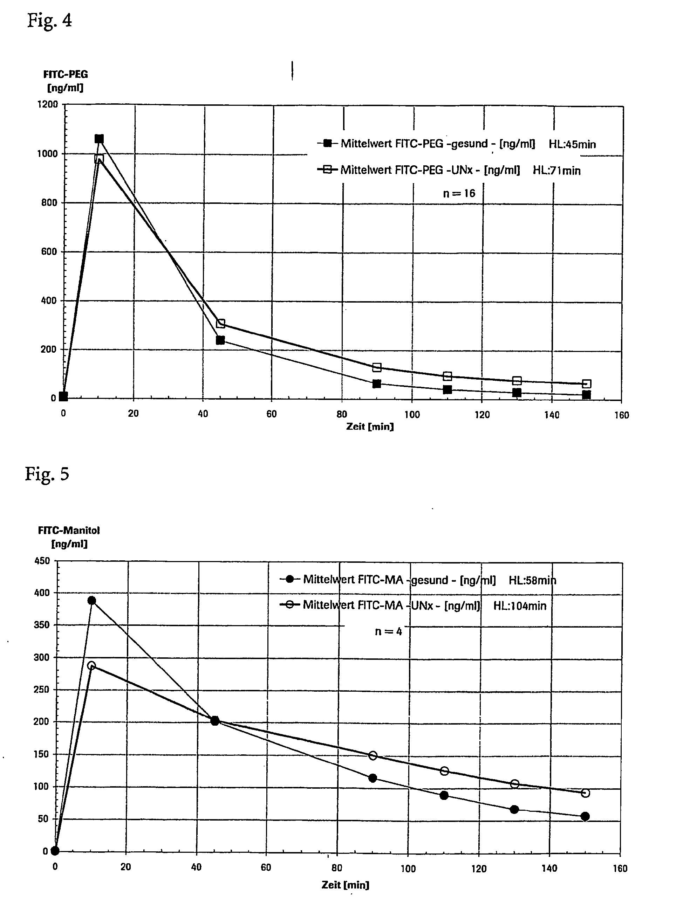

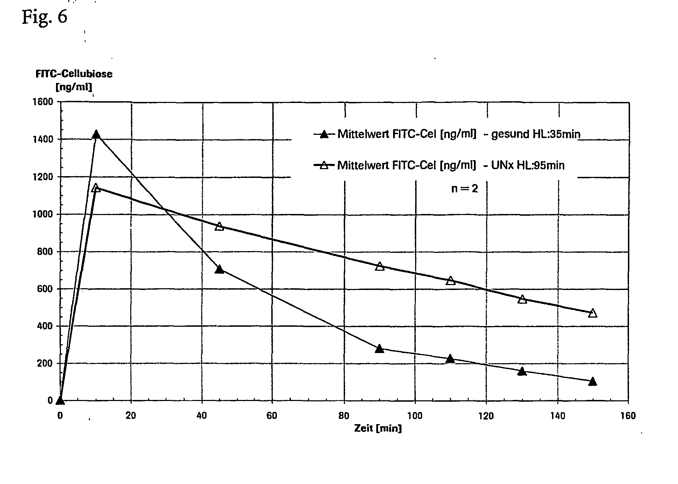

Eine Genehmigung der durchzufuhrenden Studien durch das Regierungspräsidium Karlsruhe lag vor. Die Bestimmung der GFR wurde an männlichen Sprague Dawley Ratten mit einem Köpergewicht zwischen 300 und 500 g durchgeführt. Die zu untersuchenden Substanzen, gelöst in phosphatgepufferter Kochsalzlösung, wurden über einen Dauerkatheter in die Vena jugularis appliziert. Blut zur Bestimmung der Substanzkonzentration wurde zu den in den Figuren 4 bis 6 angegebenen Zeiten mittels eines Katheters aus der Arteria femozalis entnommen. Zur Überprüfung, inwieweit die Testsubstanzen eine Reduktion der glomerulären Filtrationskapazität reflektieren, wurde die GFR sowohl an sonst intakten Tieren als auch nach zweitägiger Rekonvaleszenz nach unilateraler Nephrektomie bestimmt.

**Tabelle 1: Versuchsdesign zur in vivo Prüfung von FTTC-markierten GFR-Markern**

| **Testsubstanz** | **Dosis (µg/kg KG)** |
|---|---|
| FITC-HEG | 1800 |
| FITC-MAN | 750 |
| FITC-CEL | 1500 |

### 2. Analytik

Die Messung der Konzentration der Testsubstanz wurde in Blutplasma mit einem Standardfluorimeter bei einer Wellenlänge von 485/520 nm (unter Verwendung einer Kalibrationsfunktion entsprechend der jeweiliges Testsubstanz in Rattenplasma) durchgeführt.

### 3. Ergebnisse und Diskussion

Aufgrund des stöchiometrischen 1:1-Verhältnisses von FITC-Markierung und Polyol in der erfindungsgemäßen Verbindung konnte im Vergleich zu FITC-markiertem Inulin oder Sinistrin eine retativ niedrige Dosis verabreicht werden. Während für Sinistrin bzw. FITC-Sinistrin Dosen von 100 mg/kg Körpergewicht (KG) bzw. 5 bis 50 mg/kg KG erforderlich sind liegt der Dosisbereich bei der erfindungsgemäßen Verbindung bei deutlich unter 2 mg/kg KG (bei FITC-MAN nur 750 µg/kg KG). Die Testsubstanz war im pharmakologischen und somit dem zu erwartenden Dosisbereich beim Menschen sehr gut im Wässrigen löslich. Dies führt zu einer drastischen Reduktion des Applikationsvolumens im Vergleich zu Polyfructosanen und ihren farbstoffmarkierten Derivaten. Während für Inulin und Sinistrin typischerweise 5 g Substanz in 20 ml Lösung appliziert werden müssen reichen bei der erfindungsgemäßen Substanz 100 mg Substanz in 1 bis 2 ml Lösung.

Die Testsubstanz wurden von allen Tieren gut vertragen. Klinisch konnten keine Zeichen von Nebenwirkungen sowohl akut als auch subakut beobachtet werden.

In den Figuren 4 bis 6 sind die Konzentrations-Zeitverläfe der Testsubstanz im Blutplasma wiedergegeben. Für die Testsubstanz wurde eine relativ kurze Halbwertzeit (Tabelle 2) gefunden. Dies ist kompatibel mit der geforderten guten glomerulären Filtrierbarkeit der Testsubstanz. Die Reduktion der renalen Eliminationskapazität durch unilaterale Nephrektomie führt bei der Testsubstanz zu einer deutlichen Erhöhung der Halbwertzeit und zeigt, dass die Testsubstanz prinzipiell als GFR-Marker geeignet ist. Der ca 50%ige Verlust der Filtrationskapazität wird nahezu ideal durch FITC-MAN wiedergegeben.

**Tabelle 2: Halbwertzeit (t/₂) im Blutplasma von FITC-CEL, FITC-MAN und FITC-HEG in der Ratte vor und nach unilateraler Nephrektomie (UNX)**

| Testsubstanz | t/₂ vor UNX | t/₂ nach UNX |
|---|---|---|
| FITC-HEG (Vergleich) | 45 ± 16 min | 71 ± 39 min |
| FITC-MAN | 58 ± 7 min | 104 ± 37 min |
| FITC-CEL (Vergleich) | 34 ± 6 min | 95 ± 12 min |

## Patentansprüche

1. Verbindungen der Formel I
P-Lₘ-F (I)
in der
P ein Polyol ist, das aus der Gruppe ausgewählt ist umfassend Glycerin, Mannitol, Sorbitol, Hexite, Pentite, Tetrite, Inosite, Mannose, Aldosen, Laktose, Gentiobiose, β-Alkylglykoside, Desoxyzucker, β-Alkyluronsäuren, Fucose, Desoxyzuckeralkohofe, wobei das Polyol gegebenenfalls auch als Desoxyaminozuckeralkohol vorliegen kann,
L eine Linkergruppe ist, die aus der Gruppe ausgewählt ist, bestehend aus Thionamstoff-Gruppe (-N-CS-N-), Thiocarbamat-Gruppe (-N-CS-O-), Carbamat-(Urethan-)-Gruppe (-N-CO-O-), Ether-Gruppe (-O-), Thioether-Gruppe (-S-), Ester-Gruppe (-CO-O-), Amid-Gruppe (-CO-N-), Thioester-Gruppe (-CS-O-), Thioamid-Gruppe (-CS-N-), Aminoalkyl-Gruppe (-CO-N-(CH₂)ₙ-O-) mit n = 2 bis 5, sekundäre Amin-Gruppe (-NH-), wobei m 1 ist, und
F ein Farbstoff ist, der aus der Gruppe ausgewählt ist, umfassend Fluorescein-Farbstoffe, Cyanin-Farbstoffe, Naphthylamid-Farbstoffe, Coumarin-Farbstoffe, Xanthen-Farbstoffe, Thioxanthen-Farbstoffe, Naphtholacton-Farbstoffe, Azlacton-Farbstoffe, Methin-Farbstoffe, Oxazin-Farbstoffe, Thiazin-Farbstoffe.

2. Verbindung gemäß Anspruch 1, **dadurch gekennzeichnet, dass** das Polyol Mannose ist, wobei das Polyol gegebenenfalls auch als Desoxyaminozuckeralkohol vorliegen kann.

3. Verbindung gemäß Anspruch 1 oder 2, **dadurch gekennzeichnet, dass** der Farbstoff ein Fluoreszenzfarbstoff ist.

4. Verbindung gemäß Anspruch 3, **dadurch gekennzeichnet, dass** der Farbstoff Fluorescein ist.

5. Verwendung einer Verbindung gemäß einem der Ansprüche 1 bis 4 zur Herstellung einer diagnostischen Zubereitung für die Nierendiagnostik.

6. Diagnostische Zubereitung enthaltend eine Verbindung gemäß einem der Ansprüche 1 bis 4.

7. Verbindung gemäß einem der Ansprüche 1 bis 4 oder diagnostische Zubereitung gemäß Anspruch 6 zur Anwendung in der Messung der Nierenfunktion, umfassend das Verabreichen der Verbindung oder der diagnostischen Zubereitung und das Einstrahlen von Licht auf die Haut eines zu untersuchenden Individuums und das Erfassen des Lichts aus der Haut, in einem nicht-invasivem Verfahren.

## Claims

1. Compounds of the formula I
P-Lₘ-F (I)
where
P is a polyol which is selected from the group consisting of
glycerol, mannitol, sorbitol, hexitols, pentitols, tetritols, inositols, mannose, aldoses, lactose, gentiobiose, β-alkyl glycosides, deoxy sugars, β-alkyluronic acids, fucose, deoxy sugar alcohols, whereby the polyol can optionally also be present as a deoxyamino sugar alcohol,
L is a linker group which is selected from the group consisting of
thiourea group (-N-CS-N-), thiocarbamate group (-N-CS-O-), carbamate (urethane) group (-N-CO-O-), ether group (-O-), thioether group (-S-), ester group (-CO-O-), amide group (-CO-N-), thioester group (-CS-O-), thioamide group (-CS-N-), aminoalkyl group (-CO-N-(CH₂)ₙ-O-) where n = 2 to 5, secondary amine group (-NH-), whereby m is 1, and
F is a dye which is selected from the group consisting of
fluorescein dyes, cyanine dyes, naphthylamide dyes, coumarin dyes, xanthene dyes, thioxanthene dyes, naphtholactone dyes, azolactone dyes, methine dyes, oxazine dyes, thiazine dyes.

2. Compound according to Claim 1, **characterized in that** the polyol is mannose, wherein the polyol can optionally also be present as a deoxyamino sugar alcohol.

3. Compound according to Claim 1 or 2**, characterized in that** the dye is a fluorescence dye.

4. Compound according to Claim 3, **characterized in that** the dye is fluorescein.

5. Use of a compound according to any one of Claims 1 to 4 for producing a diagnostic formulation for kidney diagnosis.

6. Diagnostic formulation containing a compound according to any one of Claims 1 to 4.

7. Compound according to any one of Claims 1 to 4 or diagnostic formulation according to Claim 6 for use in the measurement of kidney function, comprising administering the compound or the diagnostic formulation and irradiating light onto the skin of an individual under examination and determining the light from the skin in a noninvasive method.

## Revendications

1. Composés de formule I
P-Lₘ-F (I)
dans laquelle
P est un polyol qui est choisi dans le groupe comprenant la glycérine, le mannitol, le sorbitol, l'hexite, la pentite, la tétrite, l'inosite, le mannose, les aldoses, le lactose, le gentiobiose, le β-alkylglycoside, le désoxyose, les acides β-alkyluroniques, le fucose, le désoxypolyol, le polyol pouvant aussi se présenter éventuellement sous la forme de désoxyaminopolyol,
L est un groupe de liaison qui est choisi dans le groupe comprenant le groupe thio-urée (-N-CS-N-), le groupe thiocarbamate (-N-CS-O-), le groupe carbamate-(uréthane) (-N-CO-O), le groupe éther (-0-), le groupe thioéther (-S-), le groupe ester (-CO-O-), le groupe amide (-CO-N-), le groupe thioester (-CS-O-), le groupe thioamide (-CS-N-), le groupe aminoalkyle (-CO-N-(CH₂)ₙ-O-) où n = 2 à 5, un groupe amine secondaire (-NH-) où m vaut 1 et
F est un colorant choisi dans le groupe comprenant le colorant fluorescéine, le colorant cyanine, le colorant naphtylamide, le colorant coumarine, le colorant xanthène, le colorant thioxanthène, le colorant naphtolactone, le colorant azolactone, le colorant méthine, le colorant oxazine, le colorant thiazine.

2. Composé selon la revendication 1, **caractérisé en ce que** le polyol est le mannose, le polyol pouvant aussi se présenter éventuellement sous la forme de désoxyaminopolyol.

3. Composé selon la revendication 1 ou 2, **caractérisé en ce que** le colorant est un colorant fluorescent.

4. Composé selon la revendication 3, **caractérisé en ce que** le colorant est la fluorescéine.

5. Utilisation d'un composé selon l'une des revendications 1 à 4, pour la production d'une préparation diagnostique pour le diagnostic rénal.

6. Préparation diagnostique contenant un composé selon l'une des revendications 1 à 4.

7. Composé selon l'une des revendications 1 à 4, ou préparation diagnostique selon la revendication 6, pour l'utilisation dans la mesure de la fonction rénale, comprenant l'administration du composé ou de la préparation diagnostique et l'irradiation de lumière sur la peau d'un individu à analyser et la saisie de la lumière sur la peau, dans un procédé non invasif.
